# EUROPEAN PATENT APPLICATION

(11) **EP 0 915 168 A1**
(43) Date of publication of application: **12.05.1999**
(21) Application number: 97919717.5
(22) Date of filing: 28.04.1997
(51) Int. Cl.: C12P 21/02, C12N 15/12

(54) **PROCESS FOR PRODUCING MATURATION BONE MORPHOGENETIC PROTEIN**

(30) Priority: 30.04.1996 JP 130618/96
(71) Applicant: Hoechst Marion Roussel, Ltd., Tokyo 107-8465 (JP)
(72) Inventor: TAKAHASHI, Mikiko, Kobe-shi, Hyogo 657 (JP); MAKISHIMA, F. Hoechst Marion Roussel Ltd, Kaawagoe-shi, Saitama 350-11 (JP); KIMURA, Michio Hoechst Marion Roussel Ltd, Kawagoe-shi, Saitama 350-11 (JP)
(74) Representative: Vieillefosse, Jean-Claude
(86) International application number: JP9701474
(87) International publication number: WO9741250

(57) **Abstract**

A process for producing a muturation bone morphogenetic protein by the action of a processing enzyme on a bone morphogenetic protein precursor, which comprisis either introducing an expression vector of a bone morphogenetic protein precursor and an expression vector of a processing enzyme into an animal cell strains, culturing the resultant strain to yield a maturation bone morphogenietc protein, and separating the same from the culture, or alternatively adding a solution of a processing enzyme to a solution of a bone morphogenetic protein precursor and incubating the obtained solution mixture. A suitable process comprises introducing an expression vector of a human MP52 precusor and an expression vector of a secretory furin variant into an established animal cell strain, culturing the resultant strain to yield a muturation bone morphogenetic protein, and separating the same from the culture.

## Description

### Technical Field

The present invention relates to a process for producing a maturation bone morphogenetic protein. More specifically, this invention relates to a process for producing a maturation bone morphogenetic protein, which comprises causing a processing enzyme to act on a bone morphogenetic protein precursor.

### Background of the Invention

The existence of a proteinaceous bone morphogenetic factor in the bone matrix was discovered by Urist et al. (Science, 150, pp.893-899, 1965) and it was named "bone morphogenetic protein" (which will hereinafter be abbreviated as "BMP"). In recent years, many BMP-related genes have been cloned and each of them is known to belong to the transforming growth factor-β (which will hereinafter be abbreviated as "TGF-β") super family. From some of them, recombinant proteins have already been produced. They are used for the confirmation of the bone morphogenetic activity and their application to the treatment of bone diseases is expected.

The above-described proteins belonging to the TGF-β super family are each presumed, from its gene structure, to be synthesized as a precursor *in vivo* and after subjected to various processings, form a maturation (active type) peptide homodimer. The maturation human TGF-β1 is known to be a dimer of the peptide of COOH-terminal 112 residues (Nature, 316, 701-705, 1985). In practice, it is known that in a mammalian cell, the introduction of a precursor-encoding cDNA to produce BMP-2 or BMP-6/Vgr-1 incorporates, in addition to the maturation peptide dimer, various peptide dimers (homodimers composed of large-molecular-weight monomers and heterodimers composed of a large-molecular-weight monomer and a maturation monomer) having a larger molecular weight than the maturation much in the culture supernatant (Growth Factors 7, pp.139-150,1992; J. Biol. Chem. 126, pp.1595-1609, 1994). These peptide dimers having a larger molecular weight than the maturation are presumed to correspond to the molecules under processing from the precursor to the maturation. The peptide dimer having a larger molecular weight than the maturation will hereinafter be called a "precursor dimer". It is technically very difficult to selectively produce, among the above-described various dimers, a peptide dimer of a certain molecular weight, for example, a maturation dimer on a large scale; or to carry out separation of the maturation dimer efficiently. There is accordingly a strong demand for overcoming such a technical difficulty.

### Disclosure of the Invention

An object of the present invention is to provide an efficient method for selectively producing, in a mammalian cell, a maturation dimer of the same molecular weight from the mixture of various-molecular-weight precursor dimers of a bone morphogenetic protein on a large scale.

In recent years, a group of Kex2-like proteases, furin, PC-2, PC-3, PACE4, PC6 and the like were identified as processing enzymes against the precursor of a protein in a higher animal (Biochem. J. 299, pp.1-18, 1994). Among them, furin has a hydrophobic transmembrane domain on the COOH-terminal and locally exists in the Golgi membrane. The cDNA sequence encoding human furin has already been reported by van den Ouweland et al. (Nucleic Acids Res. 18, pp.664, 1990). It has been revealed that furin recognizes Arg-X₁-X₂-Arg (wherein X₁ represents any amino acid and X₂ represents any amino acid, but mainly Lys or Arg) as a sequence of 4 amino acids upstream of its cleavage site (J. Biol. Chem. 266, pp.12127-12130, 1991). Furthermore, it has been reported that when only the NH₂-terminal domain of furin without the transmembrane domain is expressed in a mammalian cell, it is secreted outside the cell and cleavage activity specific to the amino acid sequence is maintained even at the secretion site (J. Biol. Chem. 269, pp.25830-25837, 1994).

The present inventors, for the first time, succeeded in the efficient production, in a mammalian cell, of only a maturation dimer without a precursor dimer on a large scale by using the above-described furin based on the recombinant DNA technology.

According to the present invention, a maturation bone morphogenetic protein can be produced by causing a processing enzyme to act on the bone morphogenetic protein precursor.

According to the present invention, the processing enzyme is caused to act on the bone morphogenetic protein precursor by introducing both an expression vector of the bone morphogenetic protein precursor and an expression vector of the processing enzyme into a mammalian cell strain. The cell strain is cultured and then, from the culture supernatant, the maturation bone morphogenetic protein is obtained by separation.

According to the present invention, the processing enzyme is also caused to act on the bone morphogenetic protein precursor by adding a processing-enzyme-containing culture supernatant to a culture supernatant containing bone morphogenetic protein precursors. The maturation bone morphogenetic protein is produced by incubating the resulting mixture.

Preferably, the production is carried out by introducing both an expression vector of a human MP52 precursor and an expression vector of a secretory furin mutant into a mammalian cell strain, culturing the cell strain and then, separating from the culture supernatant the maturation bone morphogenetic protein so produced.

The term "maturation bone morphogenetic protein" as used herein means a bone morphogenetic protein which is substantially composed of an amino acid sequence homologous with COOH-terminal 112 amino acid residues of active type human TGF-β1. The activity of the bone morphogenetic protein is presumed to exist in the amino acid sequence region homologous with COOH-terminal 112 amino acid residues of this active type human TGF-β1 so that the bone morphogenetic protein is desired not to contain the other amino acid sequence region.

Examples of the processing enzyme usable in the present invention include a group of Kex2-like proteases, furin, PC-2, PC-3, PACE4 and PC6. Among them, furin is particularly preferred. As cDNA which encodes furin, the DNA base sequence which encodes the whole amino acid sequence of furin can be used, but preferred is the DNA base sequence which encodes a portion of the amino acid sequence (which will hereinafter be called "secretory type furin mutant") which does not include a transmembrane domain and in addition, maintains cleavage activity specific to the amino acid sequence.

In the preferred embodiment of the present invention, a DNA fragment containing a secretory furin mutant cDNA is cloned from a total RNA extracted from a human cell strain HepG2 (ATCC HB8065) by the RT-PCR method using a synthesized DNA primer. The secretory furin mutant cDNA has, as its DNA base sequence, nucleotide No. 163 to nucleotide No. 2014 as set forth in SEQ ID No:1 of the Sequence Listing. This DNA fragment is inserted into an expression vector, whereby an expression vector of the secretory furin mutant is constructed. The expression vector to be used here contains, downstream of the eukaryote promoter, a poly(A)-added signal which is a restriction enzyme site for cloning a gene for expression. This expression vector may further contain a drug-resistant marker advantageous for the selection of a transformant. Examples of such an expression vector include human cytomegalovirus promoter enhancer, polylinker, bovine growth hormone poly(A)-added signal and pRc/CMV (available from INVITROGEN Inc.) containing a neomycin-resistant marker.

The present invention is applicable to a production method of a bone morphogenetic protein belonging to the human TGF-β super family, more specifically, to the production method of a maturation bone morphogenetic protein composed of monomers having the same molecular weight, for example, MP52, BMP-2, BMP-4, BMP-6 or BMP-7. As a bone morphogenetic protein, human MP52 which is disclosed by PCT application WO93/16099 or WO95/04819 and has bone morphogenetic activity is particularly preferred. In a preferred embodiment, cDNA which encodes a human MP52 precursor is inserted in an expression vector to construct the expression vector of the human MP52 precursor, followed by introduction into a mammalian cell, whereby the mammalian cell line which produces the precursor and the maturation MP52 dimer is prepared. Examples of the mammalian cell suited as a host cell here include Chinese hamster ovary (CHO) cell, BHK cell, 293 cell and mouse L cell. Out of them, CHO cell is preferred. Into the human-MP52-producing mammalian cell line (MC-2: deposition number FERM BP-5142) thus obtained, the expression vector of the secretory furin mutant is introduced to co-express both proteins, whereby only the maturation MP52 dimer is produced in its culture supernatant.

The conversion from the precursor protein to its maturation can also be attained by mixing a processing-enzyme-containing solution with a precursor-containing-solution and incubating the resulting mixture overnight at 32 to 40°C, preferably 37°C.

The maturation bone morphogenetic protein available by the process of the present invention can be used for the treatment or prevention of bone, cartilage or tooth injuries or for the artificial tooth root by adding to it a pharmaceutically acceptable carrier, additive, diluent and/or excipient as needed.

For the treatment of bone diseases caused by osseous dysbolism, the maturation bone morphogenetic protein can be administered in systemic through any one of the conventional manners, for example, injection such as intravenous injection, intramuscular injection or intraperitoneal injection, oral administration or parenteral administration such as suppository.

For the treatment of bone fracture, the maturation morphogenetic protein can be administered in systemic or locally by injection, oral administration or parenteral administration. It is also preferred to implant a matrix containing the maturation morphogenetic protein to a region near the fractured bone. Suitable examples of the matrix include natural polymers such as collagen or fibrin adhesive and synthetic polymers such as a copolymer of polylactic acid and glycolic acid.

In the case of orthopedic reconstruction, bone grafting or artificial tooth root, the maturation bone morphogenetic protein can be applied to the surface of the bone or tooth to be implanted, being covered with a collagen paste, fibrin adhesive or other adhesive. In the case of bone grafting, it can be used for both the natural and artificial bones.

The dose of the maturation bone morphogenetic protein is determined based on the purpose and administration method. In general, the dose is 1 µg to 100 µg/kg in the systemic administration, while it is preferably 30 µg to 30 mg/site in the local administration.

### Brief Explanation of the Drawings

FIG. 1 is a plasmid map of an expression vector, pDfurpRC/CMV (7.2kb), of a human secretory furin mutant.
FIG. 2 is a plasmid map of an expression vector, pMSS99 (5.0 kb), of human MP52.
FIG. 3 is a photo of Western blotting analysis under reducing conditions, showing the serum-free culture supernatant of a cell line which co-expresses the maturation human MP52 dimer and human secretory furin mutant.
FIG. 4 is a photo of Western blotting analysis under reducing conditions, showing that various precursor human MP52 dimers are converted into the maturation MP52 by causing the human secretory furin mutant to act on the dimers.

### Description of the preferred embodiments

The advantages of the present invention will hereinafter be described more specifically by examples.

### Example 1

### Production of the maturation human MP52 dimer by CHO cell lines which co-expresses a human secretory furin mutant and human MP52

### (1) Cloning of human secretory type furin mutant cDNA and construction of its expression vector

Human furin protein has a structure composed of a signal peptide, subtilisin-like protease domain, transmembrane domain and cytoplasm-side domain in the order of mention from the NH₂-terminal domain. In this invention, human secretory furin mutant cDNA encoding the NH₂-terminal protease domain without the transmembrane domain was cloned by the RT-PCR method and provided for expression.

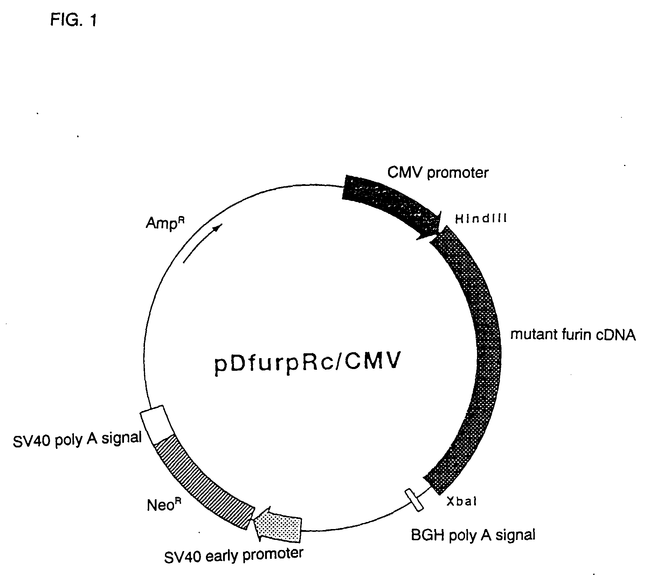

From the human HepG2 cell, total RNA was extracted and with this as a template, the upstream antisense primer 1 (human furin cDNA sequence Nos. 931 to 914 as set forth in SEQ ID No:1 of the Sequence Listing) and downstream antisense primer 2 (Nos. 2095 to 2071) were subjected to reverse transcription by the rTth RNA polymerase. These products in combination with sense primer 3 (SEQ ID No:2 of the Sequence Listing) and antisense primer 4 (SEQ ID No:3 of the Sequence Listing), and with sense primer 5 (SEQ ID No:4 of the Sequence Listing) and antisense primer 6 (SEQ ID No:5 of the Sequence Listing) were subjected to PCR reaction, respectively, whereby two cDNA fragments on the upstream side and down stream side were obtained. These fragments were joined and inserted into *Hin*dIII-*Sal*I site of plasmid pUC119 (obtained from Takara Shuzo Co., Ltd.), whereby human secretory furin mutant cDNA encoding 595 amino acids was obtained. The resulting cDNA was confirmed by the digestion with a restriction enzyme and determination of a DNA base sequence. The human secretory furin mutant cDNA was found to have, as the DNA base sequence, nucleotides Nos. 163 to 2014 of SEQ ID No:1 of the Sequence Listing. The DNA sequence of this human secretory furin mutant cDNA is different from the DNA sequence reported by van den Ouweland et al., in that base No. 165 is adenine so that an initiation codon which presumably has an adverse effect on the translation and is therefore unnecessary has been eliminated; and that base No. 2004 is adenine so that a termination codon has been formed. Then, the human secretory furin mutant cDNA was cut out by the digestion with *Hin*dIII-*Xba*I, followed by the insertion into the *Hin*dIII-*Xba*I site of the pRc/CMV vector purchased from INVITROGEN INC., whereby pDfurpRC/CMV, an expression vector of the human secretory furin mutant cDNA as illustrated in FIG. 1 was prepared.

### (2) Construction of human MP52 expression vector

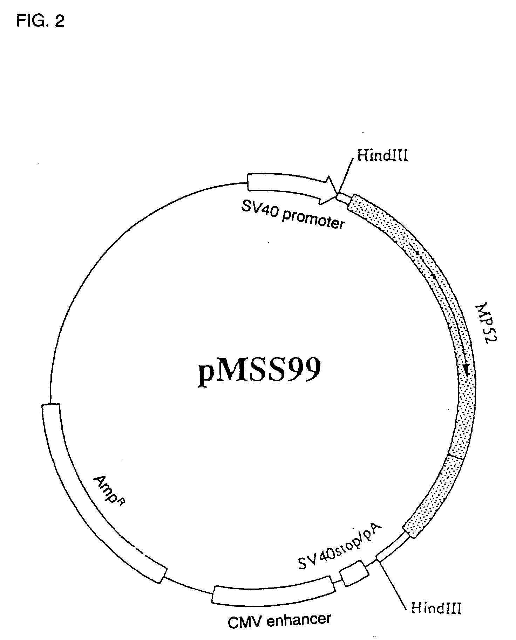

From pSK52s plasmid containing human MP52 gene provided by Dr. Hoetten of Biopharm GmbH, a DNA fragment containing the human MP52 gene was isolated by the digestion with *Hin*dIII, followed by the insertion into the *Hin*dIII site of the pABstop vector provided by Dr. Zettlmeissl of Behringwerke AG, whereby pMSS99, a human MP52 expression vector as illustrated in FIG. 2 was prepared. Its structure was confirmed by the determination of the DNA base sequence and digestion with a restriction enzyme. As a result, the human MP52 DNA base sequence of pMSS99 was found to be nucleotides Nos. 576 to 2279 of SEQ ID No:6 of the Sequence Listing.

### (3) Establishment of MC2, that is, a Chinese Hamster Ovary (CHO) cell line which produces various precursor human MP52 dimers

Into CHO-DUKX-B11 cells provided by Dr. Zettlmeissl of Behringwerke AG, that is, mutant strains of CHO cells, pMSS99 and pSVOAdhfr provided by Dr. Zettlmeissl were introduced by the calcium phosphate DNA co-precipitation method. Then, a high-production cell line of human MP52 was established by the gene amplification method using methotrexate (MTX).

pMSS99 (10 µg) and pSVOAdhfr (2 µg) were dissolved in 1 ml of 25 mM HEPES, 140 mM NaCl and 0.75 mM Na₂HPO₄ (pH 7.05) and followed by mixing with 50 µl of 2.5 M CaCl₂. The precipitate was laid over CHO-DUKX-B11 cells in a 10-cm dish and it was allowed to stand at room temperature for 30 minutes. To the cell layer, 8 ml of a ribo- and deoxyribonucleotide-containing MEM-ALPHA (MEM-α⁺) medium, containing 10% fetal calf serum were added and the resulting mixture was cultured for 4 to 6 hours in a CO₂ incubator. After the treatment with 10% glycerol at room temperature for 3 minutes, the cells were cultured on an MEM-α⁺ medium containing 10% FBS. The cultured cells were then inoculated again on a ribo- and deoxyribonucleotide-free MEM-ALPHA (MEM-α⁻) medium containing 10% dialyzed FBS and transformants were selected. The production of human MP52 was detected by Western blotting analysis as described below in (5).

In the medium of human MP52 producing cell strains, MTX was added. By increasing the concentration of the MTX successively, the cell lines having MP52 gene amplified were selected. At the MTX concentration of 400 nM, the cell line MC-2 producing precursor human MP52 dimers of various molecular weights and the maturation human MP52 dimer was obtained. The resulting cell line MC-2 was deposited in National Institute of Bioscience and Human-Technology, Agency of Industrial Science and Technology (1-3, Higashi 1-chome, Tsukuba-shi, Ibaraki-ken) on June 21, 1995 under the deposition number of FERM BP-5142.

### (4) Establishment of co-expressing cell line of human MP52 and human secretory furin mutant by the introduction of the human secretory furin mutant expression vector into the human MP52 producing CHO cell line MC-2

The co-expressing cell line of human MP52 and human secretory furin mutant was established by introducing the human secretory furin mutant expression vector into the CHO cell line MC-2 which was producing various precursor human MP52 dimers and maturation MP52 dimer by the calcium phosphate DNA co-precipitation method and then, selecting the transformant cell in the presence of 333 µg/ml of G418 and 400 nM MTX.

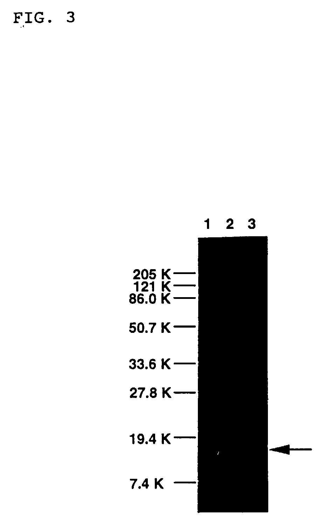

In a similar manner to Example 1(2), the phosphate calcium DNA co-precipitate was formed using pDfurpRC/CMV (4.8 µg). After laid over the MC-2 cells in a 10-cm dish, it was allowed to stand at room temperature for 30 minutes. To the cell layer, 8 ml of a ribo- and deoxyribonucleotide-free MEM-ALPHA (MEM-α⁻) medium containing 10% FBS were added and the resulting mixture was cultured for 4 to 6 hours in a CO₂ incubator. After the treatment with 10% glycerol at room temperature for 3 minutes, the cells were cultured on an MEM-α⁻ medium containing 10% FBS for 2 days. The cultured cells were then inoculated again on an MEM-α⁻medium containing 10% FBS, 400 nM MTX and 333 µg/ml G418 and transformant strains were selected. The production of human MP52 was detected by Western blotting analysis as described below in (5). The production of the human secretory furin mutant was detected by the enzyme activity measuring method as described below in (6). One of the serum-free culture supernatants of the resulting co-expression cell lines of the human MP52 furin and human secretory furin mutant was subjected to medium exchange every day and the culture supernatants collected were subjected to SDS-polyacrylamide electrophoresis under reducing conditions and followed by Western blotting analysis. Its results are shown in the photo of FIG. 3. In each lane, a 0.5 µl portion of the culture supernatant was poured. Lane 1, Lane 2 and Lane 3 are culture supernatants on Day 1, Day 2 and Day 3, respectively. The band of the maturation MP52 monomer is indicated by an arrow. It has been found that no precursor MP52 having a large molecular weight was detected in the supernatant and all the MP52s were peptides of a molecular weight of about 15K, which corresponded to the maturation MP52 monomers. As a result of an analysis under non-reducing conditions, these peptides formed the maturation MP52 dimers each having a molecular weight of about 28K. The production amount of the dimers by using the co-expressing cell line was about 8 µg/ml/24 hours, which was about three to eight times as much as that by using the MP52-expressing cell line MC-2. Thus, by using the co-expression line of human MP52 and human secretory furin mutant, the present inventors have for the first time succeeded in the production of the maturation MP52 dimers only on a larger scale than the conventional method.

### (5) Detection of human MP52 in the culture supernatant by Western blotting analysis

The proteins of the culture supernatant was separated by SDS-polyacrylamide gel electrophoresis (15-25% polyacrylamide gradient gel, Daiichi Kagaku Co., Ltd.) and followed by transfer of the protein to a PVDF membrane (Clear Blot Membrane-P, ATTO). The membrane was blocked with Block Ace (Dainippon Pharmaceutical Co., Ltd.) for one hour, rinsed with Tris buffer saline (TBS) and then treated overnight with 10 µg/ml of a chicken antibody against human MP52. The membrane was rinsed with TBS, containing 0.1% Tween 20 (TTBS) and followed by the treatment with an alkaline phosphatase-rabbit anti-chicken IgG complex (Sigma A9171). The membrane was rinsed with TTBS and the band corresponding to MP52 was visualized by the alkaline phosphatase substrate kit (BIO-RAD).

### (6) Detection of the activity of human secretory furin mutant in the culture supernatant

Twenty µl of the culture supernatant was diluted by 30 µl of pure water and mixed with 200 µl of a fluorescent substrate solution; incubating with 125 mM MES/NaOH (pH 7.0) containing 100 µM Boc-Arg-Arg-Val-Arg-MCA (purchased from Protein Technology Institute) and 1.25 mM CaCl₂ at 37°C for 10 minutes. The fluorescence of AMC was measured at an excitation wavelength of 380 nm and emission wavelength of 450 nm. As the unit of furin activity, the activity for liberating 1 pmol of AMC per minute was defined as 1 unit (U).

### (7) Purification of maturation MP52 produced by co-expression with human secretory furin mutant and analysis of NH₂-terminal amino acid sequence

With the serum-free culture supernatant of the co-expression cell strain of the human MP52 and human secretory furin mutant, 0.1 by volume of a 0.2 M sodium phosphate buffer (pH 6.0) was mixed. The resulting mixture was applied to HiTrap SF (1 ml, Pharmacia) column equilibrated by a 20 mM sodium phosphate buffer (pH 6.0) containing 50 mM NaCl, followed by washing with the same buffer. The protein was then eluted with 0.1 M sodium phosphate buffer (pH 6.0), containing 6 M guanidinohydrochloric acid. The eluate was applied to reverse-phase HPLC column Resource RPC (3 ml, Pharmacia) and the protein was eluted with 25 to 55% acetonitrile. The fraction containing maturation MP52 was subjected to NH₂-terminal amino acid sequence analysis by a pulse liquid-gas phase sequencer (Applied Biosystems model 476). The results are shown in Table 1.

**Table 1**

| Cycle | Amino acid sequence 1 | (pmol) | Amino acid sequence 2 | (pmol) |
|---|---|---|---|---|
| 1 | Arg | 11.95 | Ala | 25.51 |
| 2 | Ala | 28.75 | Pro | 14.75 |
| 3 | Pro | 17.55 | Leu | 18.07 |
| 4 | Leu | 16.47 | Ala | 14.46 |
| 5 | Ala | 16.99 | Thr | 5.02 |
| 6 | Thr | 7.15 | Arg | 7.38 |
| 7 | Arg | 9.21 | Gln | 9.08 |
| 8 | Gln | 9.54 | Gly | 13.23 |
| 9 | Gly | 11.29 | Lys | 5.29 |
| 10 | Lys | 8.04 | Arg | 6.52 |

From Table 1, it was presumed that the amino acid sequence 1 and amino acid sequence 2 were derived from the amino acid sequence from Arg 354 and that from Ala 355 of SEQ ID No: 6 of the Sequence Listing, respectively and that their molar ratio was about 1:1.

### Example 2

Conversion from human MP52 precursor dimer to maturation dimer by human secretory furin mutant

### (1) Establishment of CHO cell line which produces human secretory furin mutant

Into CHO-DUKX-B11 cells provided from Dr. Zettlmeissl of Behringwerke AG, the human secretory furin mutant expression vector, pDfurpRC/CMV, as described in Example 1(1) was introduced by the calcium phosphate DNA co-precipitation method. The transformant cells were selected in the presence of G418, whereby a high expression strain of human secretory furin mutant was established.

In a similar manner to that described in Example 1(3), the co-precipitate of pDfurpRC/CMV and calcium phosphate was prepared, laid over the CHO-DUKX-B11 cells in a 10-cm dish and allowed to stand at room temperature for 30 minutes. To the cell layer, 8 ml of a ribo- and deoxyribonucleotide-containing MEM-ALPHA (MEM-α⁺) medium containing 10% FBS were added, followed by cultivation in a CO₂ incubator for 4 to 6 hours. After the treatment of the cells with 10% glycerol at room temperature for 3 minutes, they were inoculated again on an MEM-α⁺ medium containing 10% FBS and 400 µg/ml of G418, whereby the transformant was selected. The cell line producing the human secretory furin mutant was detected by the enzyme activity measuring method as described above in Example 1(6) and the cell lines having furin activity of 500 to 1000 U/ml/24 hours were obtained.

### (2) Conversion of human precursor MP52 dimer to maturation by human secretory furin mutant

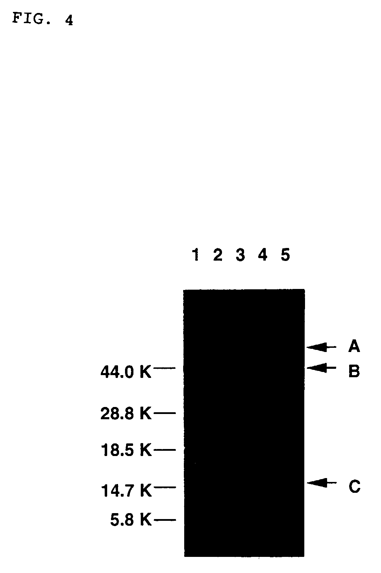

With a serum-free culture supernatant (containing the precursor and maturation MP52 dimers) of human MP-52-producing CHO cell lines MC-2, a serum-free culture supernatant (1000 U/ml) of the human secretory furin mutant expression cell line was mixed at various ratios, followed by incubating overnight at 37°C. After the reaction, Western blotting analysis as described above in Example 1(5) was carried out under reducing conditions and the conversion of the precursor MP52 to the maturation was detected. The results are shown in the photo of FIG. 4. In each of Lanes 1 to 5, a 1 µl portion of the culture supernatant of MC-2 and various volumes of the culture supernatant of the human secretory furin mutant expression cell line was poured. The final concentrations of the human secretory furin mutant were 0 U/ml for Lane 1, 50 U/ml for Lane 2, 100 U/ml for Lane 3, 200 U/ml for Lane 4 and 400 U/ml for Lane 5, respectively. The band of the maturation MP52 monomer is indicated by Arrows A and B, while that of the maturation MP52 monomer is indicated by Arrow C. As illustrated in FIG. 4, the addition of the culture supernatant of the cell line expressing the human secretory furin mutant so as to give a final active concentration of at least 200 U/ml converted MP52 to the maturation completely. As a result, the amount of the maturation MP52 dimer increased by about 3 times.

### Industrially Applicability

Maturation bone morphogenetic protein has conventionally been obtained by separating it from a mixture of dimers of bone morphogenetic proteins which are produced by mammalian cells and have various molecular weights, but it is difficult to selectively produce the maturation bone morphogenetic protein on a large scale or to obtain the maturation bone morphogenetic protein efficiently because a technology for separating it efficiently from the above-described mixture has not yet been developed. The process of the present invention, however, makes it possible to prepare the maturation bone morphogenetic protein from bone morphogenetic protein precursor, thereby facilitating the production of it on a large scale. Furthermore, the maturation bone morphogenetic protein available by the process of the present invention is a highly-pure substance composed of peptides of substantially the same molecular weight so that it is particularly suitable for use as pharmaceuticals.

"Sequence Listing"
SEQ ID NO:1
LENGTH:4180
TYPE:amino acid
STRANDEDNESS:double
TOPOLOGY:linear
MOLECULE TYPE:peptide
FRAGMENT TYPE:
ORIGINAL SOURCE:
ORGANISM:homo sapiens
TISSUE TYPE:human hepatoma
FEATURE:
LOCATION:
OTHER INFORMATION:an processing enzyme furin
SEQUENCE DESCRIPTION:SEQ ID NO:1:

GCGGGGAAGC AGCAGCGGCC AGGATGAATC CCAGGTGCTC TGGAGCTGGA TGGTGAAGGT 60

CGGCACTCTT CACCCTCCCG AGCCCTGCCC GTCTCGGCCC CATGCCCCCA CCAGTCAGCC 120

CCGGGCCACA GGCAGTGAGC AGGCACCTGG GAGCCGAGGC CCTAAGACCA GGCCAAGGAG 180

ACGGGCGCTC CAGGGTCCCA GCCACCTGTC CCCCCC ATG GAG CTG AGG CCC TGG 234

Met Glu Leu Arg Pro Trp

1               5

TTG CTA TGG GTG GTA GCA GCA ACA GGA ACC TTG GTC CTG CTA GCA GCT 282

Leu Leu Trp Val Val Ala Ala Thr Gly Thr Leu Val Leu Leu Ala Ala

            10                  15                  20

GAT GCT CAG GGC CAG AAG GTC TTC ACC AAC ACG TGG GCT GTG CGC ATC 330

Asp Ala Gln Gly Gln Lys Val Phe Thr Asn Thr Trp Ala Val Arg Ile

        25                  30                  35

CCT GGA GGC CCA GCG GTG GCC AAC AGT GTG GCA CGG AAG CAT GGG TTC 378

Pro Gly Gly Pro Ala Val Ala Asn Ser Val Ala Arg Lys His Gly Phe

    40                  45                  50

CTC AAC CTG GGC CAG ATC TTC GGG GAC TAT TAC CAC TTC TGG CAT CGA 426

Leu Asn Leu Gly Gln Ile Phe Gly Asp Tyr Tyr His Phe Trp His Arg
55                  60                  65                  70
GGA GTG ACG AAG CGG TCC CTG TCG CCT CAC CGC CCG CGG CAC AGC CGG 474
Gly Val Thr Lys Arg Ser Leu Ser Pro His Arg Pro Arg His Ser Arg
                75                  80                  85
CTG CAG AGG GAG CCT CAA GTA CAG TGG CTG GAA CAG CAG GTG GCA AAG 522
Leu Gln Arg Glu Pro Gln Val Gln Trp Leu Glu Gln Gln Val Ala Lys
            90                  95                  100
CGA CGG ACT AAA CGG GAC GTG TAC CAG GAG CCC ACA GAC CCC AAG TTT 570
Arg Arg Thr Lys Arg Asp Val Tyr Gln Glu Pro Thr Asp Pro Lys Phe
        105                 110                 115
CCT CAG CAG TGG TAC CTG TCT GGT GTC ACT CAG CGG GAC CTG AAT GTG 618
Pro Gln Gln Trp Tyr Leu Ser Gly Val Thr Gln Arg Asp Leu Asn Val
    120                 125                 130
AAG GCG GCC TGG GCG CAG GGC TAC ACA GGG CAC GGC ATT GTG GTC TCC 666
Lys Ala Ala Trp Ala Gln Gly Tyr Thr Gly His Gly Ile Val Val Ser
135                 140                 145                 150
ATT CTG GAC GAT GGC ATC GAG AAG AAC CAC CCG GAC TTG GCA GGC AAT 714
Ile Leu Asp Asp Gly Ile Glu Lys Asn His Pro Asp Leu Ala Gly Asn
                155                 160                 165
TAT GAT CCT GGG GCC AGT TTT GAT GTC AAT GAC CAG GAC CCT GAC CCC 762
Tyr Asp Pro Gly Ala Ser Phe Asp Val Asn Asp Gln Asp Pro Asp Pro
            170                 175                 180
CAG CCT CGG TAC ACA CAG ATG AAT GAC AAC AGG CAC GGC ACA CGG TGT 810

Gln Pro Arg Tyr Thr Gln Met Asn Asp Asn Arg His Gly Thr Arg Cys

        185                 190                 195

GCG GGG GAA GTG GCT GCG GTG GCC AAC AAC GGT GTC TGT GGT GTA GGT 858

Ala Gly Glu Val Ala Ala Val Ala Asn Asn Gly Val Cys Gly Val Gly

    200                 205                 210

GTG GCC TAC AAC GCC CGC ATT GGA GGG GTG CGC ATG CTG GAT GGC GAG 906

Val Ala Tyr Asn Ala Arg Ile Gly Gly Val Arg Met Leu Asp Gly Glu

215                 220                 225                 230

GTG ACA GAT GCA GTG GAG GCA CGC TCG CTG GGC CTG AAC CCC AAC CAC 954

Val Thr Asp Ala Val Glu Ala Arg Ser Leu Gly Leu Asn Pro Asn His

                235                 240                 245

ATC CAC ATC TAC AGT GCC AGC TGG GGC CCC GAG GAT GAC GGC AAG ACA 1002

Ile His Ile Tyr Ser Ala Ser Trp Gly Pro Glu Asp Asp Gly Lys Thr

            250                 255                 260

GTG GAT GGG CCA GCC CGC CTC GCC GAG GAG GCC TTC TTC CGT GGG GTT 1050

Val Asp Gly Pro Ala Arg Leu Ala Glu Glu Ala Phe Phe Arg Gly Val

        265                 270                 275

AGC CAG GGC CGA GGG GGG CTG GGC TCC ATC TTT GTC TGG GCC TCG GGG 1098

Ser Gln Gly Arg Gly Gly Leu Gly Ser Ile Phe Val Trp Ala Ser Gly

    280                 285                 290

AAC GGG GGC CGG GAA CAT GAC AGC TGC AAC TGC GAC GGC TAC ACC AAC 1146

Asn Gly Gly Arg Glu His Asp Ser Cys Asn Cys Asp Gly Tyr Thr Asn

295                 300                 305                 310

AGT ATC TAC ACG CTG TCC ATC AGC AGC GCC ACG CAG TTT GGC AAC GTG 1194

Ser Ile Tyr Thr Leu Ser Ile Ser Ser Ala Thr Gln Phe Gly Asn Val
                315                 320                 325
CCG TGG TAC AGC GAG GCC TGC TCG TCC ACA CTG GCC ACG ACC TAC AGC 1242
Pro Trp Tyr Ser Glu Ala Cys Ser Ser Thr Leu Ala Thr Thr Tyr Ser
            330                 335                 340
AGT GGC AAC CAG AAT GAG AAG CAG ATC GTG ACG ACT GAC TTG CGG CAG 1290
Ser Gly Asn Gln Asn Glu Lys Gln Ile Val Thr Thr Asp Leu Arg Gln
        345                 350                 355
AAG TGC ACG GAG TCT CAC ACG GGC ACC TCA GCC TCT GCC CCC TTA GCA 1338
Lys Cys Thr Glu Ser His Thr Gly Thr Ser Ala Ser Ala Pro Leu Ala
    360                 365                 370
GCC GGC ATC ATT GCT CTC ACC CTG GAG GCC AAT AAG AAC CTC ACA TGG 1386
Ala Gly Ile Ile Ala Leu Thr Leu Glu Ala Asn Lys Asn Leu Thr Trp
375                 380                 385                 390
CGG GAC ATG CAA CAC CTG GTG GTA CAG ACC TCG AAG CCA GCC CAC CTC 1434
Arg Asp Met Gln His Leu Val Val Gln Thr Ser Lys Pro Ala His Leu
                395                 400                 405
AAT GCC AAC GAC TGG GCC ACC AAT GGT GTG GGC CGG AAA GTG AGC CAC 1482
Asn Ala Asn Asp Trp Ala Thr Asn Gly Val Gly Arg Lys Val Ser His
            410                 415                 420
TCA TAT GGC TAC GGG CTT TTG GAC GCA GGC GCC ATG GTG GCC CTG GCC 1530
Ser Tyr Gly Tyr Gly Leu Leu Asp Ala Gly Ala Met Val Ala Leu Ala
        425                 430                 435
CAG AAT TGG ACC ACA GTG GCC CCC CAG CGG AAG TGC ATC ATC GAC ATC 1578

Gln Asn Trp Thr Thr Val Ala Pro Gln Arg Lys Cys Ile Ile Asp Ile
    440                 445                 450
CTC ACC GAG CCC AAA GAC ATC GGG AAA CGG CTC GAG GTG CGG AAG ACC 1626
Leu Thr Glu Pro Lys Asp Ile Gly Lys Arg Leu Glu Val Arg Lys Thr
455                 460                 465                 470
GTG ACC GCG TGC CTG GGC GAG CCC AAC CAC ATC ACT CGG CTG GAG CAC 1674
Val Thr Ala Cys Leu Gly Glu Pro Asn His Ile Thr Arg Leu Glu His
                475                 480                 485
GCT CAG GCG CGG CTC ACC CTG TCC TAT AAT CGC CGT GGC GAC CTG GCC 1722
Ala Gln Ala Arg Leu Thr Leu Ser Tyr Asn Arg Arg Gly Asp Leu Ala
            490                 495                 500
ATC CAC CTG GTC AGC CCC ATG GGC ACC CGC TCC ACC CTG CTG GCA GCC 1770
Ile His Leu Val Ser Pro Met Gly Thr Arg Ser Thr Leu Leu Ala Ala
        505                 510                 515
AGG CCA CAT GAC TAC TCC GCA GAT GGG TTT AAT GAC TGG GCC TTC ATG 1818
Arg Pro His Asp Tyr Ser Ala Asp Gly Phe Asn Asp Trp Ala Phe Met
    520                 525                 530
ACA ACT CAT TCC TGG GAT GAG GAT CCC TCT GGC GAG TGG GTC CTA GAG 1866
Thr Thr His Ser Trp Asp Glu Asp Pro Ser Gly Glu Trp Val Leu Glu
535                 540                 545                 550
ATT GAA AAC ACC AGC GAA GCC AAC AAC TAT GGG ACG CTG ACC AAG TTC 1914
Ile Glu Asn Thr Ser Glu Ala Asn Asn Tyr Gly Thr Leu Thr Lys Phe
                555                 560                 565
ACC CTC GTA CTC TAT GGC ACC GCC CCT GAG GGG CTG CCC GTA CCT CCA 1962

Thr Leu Val Leu Tyr Gly Thr Ala Pro Glu Gly Leu Pro Val Pro Pro

            570                 575                 580

GAA AGC AGT GGC TGC AAG ACC CTC ACG TCC AGT CAG GCC TGA 2004

Glu Ser Ser Gly Cys Lys Thr Leu Thr Ser Ser Gln Ala ***

        585                 590                 595

GTGGTGTGCG AGGAAGGCTT CTCCCTGCAC CAGAAGAGCT GTGTCCAGCA CTGCCCTCCA 2064

GGCTTCGCCC CCCAAGTCCT CGATACGCAC TATAGCACCG AGAATGACGT GGAGACCATC 2124

CGGGCCAGCG TCTGCGCCCC CTGCCACGCC TCATGTGCCA CATGCCAGGG GCCGGCCCTG 2184

ACAGACTGCC TCAGCTGCCC CAGCCACGCC TCCTTGGACC CTGTGGAGCA GACTTGCTCC 2244

CGGCAAAGCC AGAGCAGCCG AGAGTCCCCG CCACAGCAGC AGCCACCTCG GCTGCCCCCG 2304

GAGGTGGAGG CGGGGCAACG GCTGCGGGCA GGGCTGCTGC CCTCACACCT GCCTGAGGTG 2364

GTGGCCGGCC TCAGCTGCGC CTTCATCGTG CTGGTCTTCG TCACTGTCTT CCTGGTCCTG 2424

CAGCTGCGCT CTGGCTTTAG TTTTCGGGGG GTGAAGGTGT ACACCATGGA CCGTGGCCTC 2484

ATCTCCTACA AGGGGCTGCC CCCTGAAGCC TGGCAGGAGG AGTGCCCGTC TGACTCAGAA 2544

GAGGACGAGG GCCGGGGCGA GAGGACCGCC TTTATCAAAG ACCAGAGCGC CCTCTGATGA 2604

GCCCACTGCC CACCCCCTCA AGCCAATCCC CTCCTTGGGC ACTTTTTAAT TCACCAAAGT 2664

ATTTTTTTAT CTTGGGACTG GGTTTGGACC CCAGCTGGGA GGCAAGAGGG GTGGAGACTG 2724

TTTCCCATCC TACCCTCGGG CCCACCTGGC CACCTGAGGT GGGCCCAGGA CCAGCTGGGG 2784

CGTGGGGAGG GCCGTACCCC ACCCTCAGCA CCCCTTCCAT GTGGAGAAAG GAGTGAAACC 2844

TTTAGGGCAG CTTGCCCCGG CCCCGGCCCC AGCCAGAGTT CCTGCGGAGT GAAGAGGGGC 2904

AGCCCTTGCT TGTTGGGATT CCTGACCCAG GCCGCAGCTC TTGCCCTTCC CTGTCCCTCT 2964

AAAGCAATAA TGGTCCCATC CAGGCAGTCG GGGGCTGGCC TAGGAGATAT CTGAGGGAGG 3024

AGGCCACCTC TCCAAGGGCT TCTGCACCCT CCACCCTGTC CCCCAGCTCT GGTGAGTCTT 3084

GGCGGCAGCA GCCATCATAG GAAGGGACCA AGGCAAGGCA GGTGCCTCCA GGTGTGCACG 3144

TGGCATGTGG CCTGTGGCCT GTGTCCCATG ACCCACCCCT GTGCTCCGTG CCTCCACCAC 3204

CACTGGCCAC CAGGCTGGCG CAGCCAAGGC CGAAGCTCTG GCTGAACCCT GTGCTGGTGT 3264

CCTGACCACC CTCCCCTCTC TTGCACCCGC CTCTCCCGTC AGGGCCCAAG TCCCTGTTTT 3324

CTGAGCCCGG GCTGCCTGGG CTGTTGGCAC TCACAGACCT GGAGCCCCTG GGTGGGTGGT 3384

GGGGAGGGGC GCTGGCCCAG CCGGCCTCTC TGGCCTCCCA CCCGATGCTG CTTTCCCCTG 3444

TGGGGATCTC AGGGGCTGTT TGAGGATATA TTTTCACTTT GTGATTATTT CACTTTAGAT 3504

GCTGATGATT TGTTTTTGTA TTTTTAATGG GGGTAGCAGC TGGACTACCC ACGTTCTCAC 3564

ACCCACCGTC CGCCCTGCTC CTCCCTGGCT GCCCTGGCCC TGAGGTGTGG GGGCTGCAGC 3624

ATGTTGCTGA GGAGTGAGGA ATAGTTGAGC CCCAAGTCCT GAAGAGGCGG GCCAGCCAGG 3684

CGGGCTCAAG GAAAGGGGGT CCCAGTGGGA GGGGCAGGCT GACATCTGTG TTTCAAGTGG 3744

GGCTCGCCAT GCCGGGGGTT CATAGGTCAC TGGCTCTCCA AGTGCCAGAG GTGGGCAGGT 3804

GGTGGCACTG AGCCCCCCCA ACACTGTGCC CTGGTGGAGA AAGCACTGAC CTGTCATGCC 3864

CCCCTCAAAC CTCCTCTTCT GACGTGCCTT TTGCACCCCT CCCATTAGGA CAATCAGTCC 3924

CCTCCCATCT GGGAGTCCCC TTTTCTTTTC TACCCTAGCC ATTCCTGGTA CCCAGCCATC 3984

TGCCCAGGGG TGCCCCCTCC TCTCCCATCC CCCTGCCCTC GTGGCCAGCC CGGCTGGTTT 4044

TGTAAGATAC TGGGTTGGTG CACAGTGATT TTTTTCTTGT AATTTAAACA GGCCCAGCAT 4104

TGCTGGTTCT ATTTAATGGA CATGAGATAA TGTTAGAGGT TTTAAAGTGA TTAAACGTGC 4164

AGACTATGCA AACCAG 4180

SEQ ID NO:2
LENGTH:29
TYPE:nucleic acid
STRANDEDNESS:single
TOPOLOGY:linear
MOLECULE TYPE:other nucleic acid
ORIGINAL SOURCE:no
  ORGANISM:no
  STRAIN:no
FEATURE: furin's sense primer 3
SEQUENCE DESCRIPTION:SEQ ID NO:2:

GCGAAGCTTA AGACCAGGCC AAGGAGACG 29

SEQ ID NO:3
LENGTH:26
TYPE:nucleic acid
STRANDEDNESS:single
TOPOLOGY:linear
MOLECULE TYPE:other nucleic acid
ORIGINAL SOURCE:no
  ORGANISM:no
  STRAIN:no
FEATURE: furin's anti-sense primer 4
SEQUENCE DESCRIPTION:SEQ ID NO:3:
CCTCGCCATC CAGCATGCGC ACCCCT 26

SEQ ID NO:4
LENGTH:26
TYPE:nucleic acid
STRANDEDNESS:single
TOPOLOGY:linear
MOLECULE TYPE:other nucleic acid
ORIGINAL SOURCE:no
  ORGANISM:no
  STRAIN:no
FEATURE: furin's sense primer 5
SEQUENCE DESCRIPTION:SEQ ID NO:4:
TGGAGGGGTG CGCATGCTGG ATGGCG 26

SEQ ID NO:5
LENGTH:30
TYPE:nucleic acid
STRANDEDNESS:single
TOPOLOGY:linear
MOLECULE TYPE:other nucleic acid
ORIGINAL SOURCE:no
  ORGANISM:no
  STRAIN:no
FEATURE: furin's anti-sense primer 6
SEQUENCE DESCRIPTION:SEQ ID NO:5:
GGCGTCGACG CACACCACTC AGGCCTGACT 30

SEQ ID NO:6
LENGTH:2703
TYPE:amino acid
STRANDEDNESS:double
TOPOLOGY:linear
MOLECULE TYPE:peptide
FRAGMENT TYPE:

ORIGINAL SOURCE:
  ORGANISM:homo sapiens
  TISSUE TYPE:human embryo
FEATURE:
  LOCATION:
  OTHER INFORMATION:bone morphogenetic protein MP52
SEQUENCE DESCRIPTION:SEQ ID NO:6:

CCATGGCCTC GAAAGGGCAG CGGTGATTTT TTTCACATAA ATATATCGCA CTTAAATGAG 60

TTTAGACAGC ATGACATCAG AGAGTAATTA AATTGGTTTG GGTTGGAATT CCGTTTCCAA 120

TTCCTGAGTT CAGGTTTGTA AAAGATTTTT CTGAGCACCT GCAGGCCTGT GAGTGTGTGT 180

GTGTGTGTGT GTGTGTGTGT GTGTGTGTGA AGTATTTTCA CTGGAAAGGA TTCAAAACTA 240

GGGGGAAAAA AAAACTGGAG CACACAGGCA GCATTACGCC ATTCTTCCTT CTTGGAAAAA 300

TCCCTCAGCC TTATACAAGC CTCCTTCAAG CCCTCAGTCA GTTGTGCAGG AGAAAGGGGG 360

CGGTTGGCTT TCTCCTTTCA AGAACGAGTT ATTTTCAGCT GCTGACTGGA GACGGTGCAC 420

GTCTGGATAC GAGAGCATTT CCACTATGGG ACTGGATACA AACACACACC CGGCAGACTT 480

CAAGAGTCTC AGACTGAGGA GAAAGCCTTT CCTTCTGCTG CTACTGCTGC TGCCGCTGCT 540

TTTGAAAGTC CACTCCTTTC ATGGTTTTTC CTGCCAAACC AGAGGCACCT TTGCTGCTGC 600

CGCTGTTCTC TTTGGTGTCA TTCAGCGGCT GGCCAGAGG ATG AGA CTC CCC AAA 654

Met Arg Leu Pro Lys

-25

CTC CTC ACT TTC TTG CTT TGG TAC CTG GCT TGG CTG GAC CTG GAA TTC 702

Leu Leu Thr Phe Leu Leu Trp Tyr Leu Ala Trp Leu Asp Leu Glu Phe

-20 -15 -10

ATC TGC ACT GTG TTG GGT GCC CCT GAC TTG GGC CAG AGA CCC CAG GGG 750

Ile Cys Thr Val Leu Gly Ala Pro Asp Leu Gly Gln Arg Pro Gln Gly

-5 1 5 10

ACC AGG CCA GGA TTG GCC AAA GCA GAG GCC AAG GAG AGG CCC CCC CTG 798

Thr Arg Pro Gly Leu Ala Lys Ala Glu Ala Lys Glu Arg Pro Pro Leu
15 20 25
GCC CGG AAC GTC TTC AGG CCA GGG GGT CAC AGC TAT GGT GGG GGG GCC 846
Ala Arg Asn Val Phe Arg Pro Gly Gly His Ser Tyr Gly Gly Gly Ala
30 35 40
ACC AAT GCC AAT GCC AGG GCA AAG GGA GGC ACC GGG CAG ACA GGA GGC 894
Thr Asn Ala Asn Ala Arg Ala Lys Gly Gly Thr Gly Gln Thr Gly Gly
45 50 55
CTG ACA CAG CCC AAG AAG GAT GAA CCC AAA AAG CTG CCC CCC AGA CCG 942
Leu Thr Gln Pro Lys Lys Asp Glu Pro Lys Lys Leu Pro Pro Arg Pro
60 65 70
GGC GGC CCT GAA CCC AAG CCA GGA CAC CCT CCC CAA ACA AGG CAG GCT 990
Gly Gly Pro Glu Pro Lys Pro Gly His Pro Pro Gln Thr Arg Gln Ala
75 80 85 90
ACA GCC CGG ACT GTG ACC CCA AAA GGA CAG CTT CCC GGA GGC AAG GCA 1038
Thr Ala Arg Thr Val Thr Pro Lys Gly Gln Leu Pro Gly Gly Lys Ala
95 100 105
CCC CCA AAA GCA GGA TCT GTC CCC AGC TCC TTC CTG CTG AAG AAG GCC 1086
Pro Pro Lys Ala Gly Ser Val Pro Ser Ser Phe Leu Leu Lys Lys Ala
110 115 120
AGG GAG CCC GGG CCC CCA CGA GAG CCC AAG GAG CCG TTT CGC CCA CCC 1134
Arg Glu Pro Gly Pro Pro Arg Glu Pro Lys Glu Pro Phe Arg Pro Pro
125 130 135
CCC ATC ACA CCC CAC GAG TAC ATG CTC TCG CTG TAC AGG ACG CTG TCC 1182

Pro Ile Thr Pro His Glu Tyr Met Leu Ser Leu Tyr Arg Thr Leu Ser

140 145 150

GAT GCT GAC AGA AAG GGA GGC AAC AGC AGC GTG AAG TTG GAG GCT GGC 1230

Asp Ala Asp Arg Lys Gly Gly Asn Ser Ser Val Lys Leu Glu Ala Gly

155 160 165 170

CTG GCC AAC ACC ATC ACC AGC TTT ATT GAC AAA GGG CAA GAT GAC CGA 1278

Leu Ala Asn Thr Ile Thr Ser Phe Ile Asp Lys Gly Gln Asp Asp Arg

175 180 185

GGT CCC GTG GTC AGG AAG CAG AGG TAC GTG TTT GAC ATT AGT GCC CTG 1326

Gly Pro Val Val Arg Lys Gln Arg Tyr Val Phe Asp Ile Ser Ala Leu

190 195 200

GAG AAG GAT GGG CTG CTG GGG GCC GAG CTG CGG ATC TTG CGG AAG AAG 1374

Glu Lys Asp Gly Leu Leu Gly Ala Glu Leu Arg Ile Leu Arg Lys Lys

205 210 215

CCC TCG GAC ACG GCC AAG CCA GCG GCC CCC GGA GGC GGG CGG GCT GCC 1422

Pro Ser Asp Thr Ala Lys Pro Ala Ala Pro Gly Gly Gly Arg Ala Ala

220 225 230

CAG CTG AAG CTG TCC AGC TGC CCC AGC GGC CGG CAG CCG GCC TCC TTG 1470

Gln Leu Lys Leu Ser Ser Cys Pro Ser Gly Arg Gln Pro Ala Ser Leu

235 240 245 250

CTG GAT GTG CGC TCC GTG CCA GGC CTG GAC GGA TCT GGC TGG GAG GTG 1518

Leu Asp Val Arg Ser Val Pro Gly Leu Asp Gly Ser Gly Trp Glu Val

255 260 265

TTC GAC ATC TGG AAG CTC TTC CGA AAC TTT AAG AAC TCG GCC CAG CTG 1566

Phe Asp Ile Trp Lys Leu Phe Arg Asn Phe Lys Asn Ser Ala Gln Leu
270 275 280
TGC CTG GAG CTG GAG GCC TGG GAA CGG GGC AGG GCC GTG GAC CTC CGT 1614
Cys Leu Glu Leu Glu Ala Trp Glu Arg Gly Arg Ala Val Asp Leu Arg
285 290 295
GGC CTG GGC TTC GAC CGC GCC GCC CGG CAG GTC CAC GAG AAG GCC CTG 1662
Gly Leu Gly Phe Asp Arg Ala Ala Arg Gln Val His Glu Lys Ala Leu
300 305 310
TTC CTG GTG TTT GGC CGC ACC AAG AAA CGG GAC CTG TTC TTT AAT GAG 1710
Phe Leu Val Phe Gly Arg Thr Lys Lys Arg Asp Leu Phe Phe Asn Glu
315 320 325 330
ATT AAG GCC CGC TCT GGC CAG GAC GAT AAG ACC GTG TAT GAG TAC CTG 1758
Ile Lys Ala Arg Ser Gly Gln Asp Asp Lys Thr Val Tyr Glu Tyr Leu
335 340 345
TTC AGC CAG CGG CGA AAA CGG CGG GCC CCA CTG GCC ACT CGC CAG GGC 1806
Phe Ser Gln Arg Arg Lys Arg Arg Ala Pro Leu Ala Thr Arg Gln Gly
350 355 360
AAG CGA CCC AGC AAG AAC CTT AAG GCT CGC TGC AGT CGG AAG GCA CTG 1854
Lys Arg Pro Ser Lys Asn Leu Lys Ala Arg Cys Ser Arg Lys Ala Leu
365 370 375
CAT GTC AAC TTC AAG GAC ATG GGC TGG GAC GAC TGG ATC ATC GCA CCC 1902
His Val Asn Phe Lys Asp Met Gly Trp Asp Asp Trp Ile Ile Ala Pro
380 385 390
CTT GAG TAC GAG GCT TTC CAC TGC GAG GGG CTG TGC GAG TTC CCA TTG 1950

Leu Glu Tyr Glu Ala Phe His Cys Glu Gly Leu Cys Glu Phe Pro Leu
395 400 405 410
CGC TCC CAC CTG GAG CCC ACG AAT CAT GCA GTC ATC CAG ACC CTG ATG 1998
Arg Ser His Leu Glu Pro Thr Asn His Ala Val Ile Gln Thr Leu Met
415 420 425
AAC TCC ATG GAC CCC GAG TCC ACA CCA CCC ACC TGC TGT GTG CCC ACG 2046
Asn Ser Met Asp Pro Glu Ser Thr Pro Pro Thr Cys Cys Val Pro Thr
430 435 440
CGG CTG AGT CCC ATC AGC ATC CTC TTC ATT GAC TCT GCC AAC AAC GTG 2094
Arg Leu Ser Pro Ile Ser Ile Leu Phe Ile Asp Ser Ala Asn Asn Val
445 450 455
GTG TAT AAG CAG TAT GAG GAC ATG GTC GTG GAG TCG TGT GGC TGC AGG 2142
Val Tyr Lys Gln Tyr Glu Asp Met Val Val Glu Ser Cys Gly Cys Arg
460 465 470
TAG CAGCACTGGC CCTCTGTCTT CCTGGGTGGC ACATCCCAAG AGCCCCTTCC 2195
***
475
TGCACTCCTG GAATCACAGA GGGGTCAGGA AGCTGTGGCA GGAGCATCTA CACAGCTTGG 2255
GTGAAAGGGG ATTCCAATAA GCTTGCTCGC TCTCTGAGTG TGACTTGGGC TAAAGGCCCC 2315
CTTTTATCCA CAAGTTCCCC TGGCTGAGGA TTGCTGCCCG TCTGCTGATG TGACCAGTGG 2375
CAGGCACAGG TCCAGGGAGA CAGACTCTGA ATGGGACTGA GTCCCAGGAA ACAGTGCTTT 2435
CCGATGAGAC TCAGCCCACC ATTTCTCCTC ACCTGGGCCT TCTCAGCCTC TGGACTCTCC 2495
TAAGCACCTC TCAGGAGAGC CACAGGTGCC ACTGCCTCCT CAAATCACAT TTGTGCCTGG 2555
TGACTTCCTG TCCCTGGGAC AGTTGAGAAG CTGACTGGGC AAGAGTGGGA GAGAAGAGGA 2615

GAGGGCTTGG ATAGAGTTGA GGAGTGTGAG GCTGTTAGAC TGTTAGATTT AAATGTATAT 2675

TGATGAGATA AAAAGCAAAA CTGTGCCT 2703

## Claims

1. A process for producing a maturation bone morphogenetic protein, characterized in that a bone morphogenetic protein precursor is treated with a processing enzyme.

2. A process for producing a maturation bone morphogenetic protein which comprises the steps of:
introducing an expression vector for a bone morphogenetic protein precursor and a processing enzyme transformed into a mammalian cell;
culturing said mammalian cell cultured, whereby a maturation bone morphogenetic protein is produced; and then,
separating a maturation bone morphogenetic protein purified from the culture supernatant.

3. The process for producing a maturation bone morphogenetic protein as claimed in claim 1, wherein the maturation bone morphogenetic protein is selected from the group consisting of maturation MP52, BMP-2, BMP-4, BMP-6 and BMP-7.

4. The process for producing a maturation bone morphogenetic protein as claimed in at least one of claims 1 to 3, wherein the processing enzyme is furin.

5. The process for producing a maturation bone morphogenetic protein as claimed in at least one of claims 1 to 4, wherein the processing enzyme is furin consisting of a full amino acid sequence.

6. The process for producing a maturation bone morphogenetic protein as claimed in at least one of claims 1 to 4, wherein the processing enzyme is a secretory furin mutant.

7. The process for producing a maturation bone morphogenetic protein as claimed in at least one of claims 1 to 5, which comprises the steps of:
introducing an expression vector for a MP52 precursor and a secretory furin mutant transformed into a mammalian cell;
culturing said mammalian cell cultured, whereby a maturation bone morphogenetic protein is produced; and then,
separating a maturation bone morphogenetic protein purified from the culture supernatant.

8. A process for producing a maturation bone morphogenetic protein, which comprises the steps of:
adding a solution containing a processing enzyme to a solution containing a bone morphogenetic protein precursor, thereafter incubating the mixture.
